# EUROPEAN PATENT APPLICATION

(11) **EP 4 190 229 A1**
(43) Date of publication of application: **07.06.2023**
(21) Application number: 21851423.0
(22) Date of filing: 02.06.2021
(51) Int. Cl.: A61B 5/00

(54) **VEIN TRANSILLUMINATOR**

(30) Priority: 28.07.2020 ES 202031672 U
(71) Applicant: García Mingo, Francisco Javier, 03509 Finestrat Alicante (ES)
(72) Inventor: García Mingo, Francisco Javier, 03509 Finestrat Alicante (ES)
(74) Representative: Ungria López, Javier
(86) International application number: PCT/ES2021/070398
(87) International publication number: WO 2022/023593

(57) **Abstract**

The invention relates to a vein transilluminator comprising: a body formed by two arms, a plurality of LEDs housed in said arms and electrical power supply means configured to electrically power the LEDs; characterised in that the arms are joined together by means of at least one joint, said joint being configured in such a way as to allow said arms to be arranged in two different planes.

## Description

### Object of the invention

The present invention belongs to the technical field of medical devices and is particularly useful in the field of vision improvement in medical interventions and tests such as, by way of example and without limitation, blood extractions from patients and interventions to sclerose varicose veins.

More particularly, the purpose of the device according to the present invention is to allow correct visualization of portions of veins, arteries, or blood capillaries, which are not detectable with the naked eye.

### Background of the invention

Transillumination is a known technique in medicine that contemplates causing light radiation to strike a portion of the body, cavity, or body organ of a patient for diagnostic or vision aiding purposes, during a medical intervention or test.

Transillumination is usually performed in a room, preferably with dim lighting. In addition, to carry it out, a light is aimed at the portion of the body, cavity, or body organ of the subject of interest, and due to the slight translucency of the part considered, a portion of the incident light is able to reach it, illuminating it.

The use of various transillumination techniques on different body surfaces to detect various pathologies (hydroceles, hydrocephalus, cysts, etc.), as well as to avoid accidental intravascular punctures in aesthetic interventions by means of fillers or implants (hyaluronic type, hydroxyapatite, collagen, etc.) is known in the state of the art.

Likewise, Spanish patent application ES 2 534 762 T3 (derived from European patent no. EP 1849406) discloses a transillumination adapter intended for newborns or babies.

PCT patent application WO2012/087089 describes other examples of fiber optic-based transillumination devices specifically designed for the visualization of veins, arteries, or blood capillaries.

It is important to note that there is a group of people, made up of, among others, patients undergoing chemotherapy treatment, dialysis patients, obese people, children, the elderly, and people with dark skin, for whom locating their veins is more complicated than usual.

In view of this, there is still a need in the sector to develop new transillumination devices having an improved capability for the visualization of veins, arteries, or blood capillaries.

Furthermore, it is also desirable for said new transillumination devices be able to better adapt to the three-dimensional geometry of certain parts of the body (particularly the legs or arms), and for them to have a lighter weight and to be easier to transport than the already existing devices.

### Description of the invention

In order to achieve the objectives and avoid the drawbacks mentioned in the preceding sections, the object of the present invention relates to a vein transilluminator comprising:
- a body formed by two arms;
- a plurality of LEDs (light emitting diodes) housed in said arms;
- electrical power supply means configured to electrically power the LEDs;
characterised in that the arms are joined together by means of at least one joint, said joint being configured in such a way as to allow said arms to be arranged in two different planes.

As a result of the joint described above, the vein transilluminator according to the present invention can readily change its shape in order to adapt anatomically to the geometry of the part of the body on which it will act. The fact of being able to arrange each of the arms in a different plane allows said transilluminator to adapt particularly well to the shape of the legs or arms of the patient who is the object of the medical intervention or test.

The LEDs for use in the transilluminator of the present invention are preferably warm light LEDs. In other words, LEDs the correlated color temperature of which is of the order of 3000 K, for example, with a correlated color temperature in the range of 2700 - 3500 K).

The use of LEDs is beneficial because its energy consumption is considerably lower than that of other light sources. This enables electrically powering said LEDs by means of batteries, which confers autonomy to the vein transilluminator, making it independent of grid connections, and allowing said vein transilluminator to be fully portable. Moreover, the preferred range of correlated color temperatures, given in the preceding paragraph, is optimal for the visualization of veins, arteries, or blood capillaries.

Accordingly, the electrical power supply means preferably comprise at least one electric battery and at least one cable electrically connecting said battery with the LEDs.

In a preferred embodiment of the present invention, the hinge is provided with locking means. When actuated, said locking means allow preventing one arm of the transilluminator from moving with respect to the other.

Lastly, in another preferred embodiment of the invention, the transilluminator is further provided with a secondary joint which is configured in such a way as to allow varying the relative position of one arm with respect to the other in the same plane.

### Brief description of the figures

As a complement to the present description, and for the purpose of helping to make the technical features of the invention more readily understandable, in accordance with practical preferred exemplary embodiments thereof, a set of drawings is attached as an integral part of said description in which the following is depicted in a non-limiting, illustrative manner:
**Figure 1A****.-** This figure shows a front view of a first embodiment of a vein transilluminator according to the present invention;
**Figure 1B****.-** This figure shows a rear view of the vein transilluminator of Figure 1A partially disassembled;
**Figure 1C****.-** This figure shows the vein transilluminator of Figure 1A in an operative position on a patient's forearm;
**Figure 2A****.-** This figure shows a front view of a second embodiment of a vein transilluminator according to the present invention; and
**Figure 2B****.-** This figure shows a rear view of the vein transilluminator of Figure 2A partially disassembled.

### Reference numbers in the drawings

(1) First arm of the transilluminator;
(1a) Cover of the first arm;
(1b) Base of the first arm;
(2) Second arm of the transilluminator;
(2a) Cover of the second arm;
(2b) Base of the second arm;
(3) LEDs;
(4) Joint;
(4a) Threaded bolt;
(5) Locking means;
(5a) and (5b) Another embodiment of alternative locking means integrated in the housing (8);
(6a) Electrical connection cables;
(6b) Box with a power switch for the power supply means and control electronics;
(7) Patient's forearm;
(8) Housing for batteries in the body of the transilluminator;
(8a) Cover of the housing;
(8b) Base of the housing;
(10) Vein transilluminator;
(11) Secondary joint.

### Description of an exemplary embodiment of the invention

Throughout the present description and in the figures, elements with identical or similar functions will be denoted with the same reference numbers.

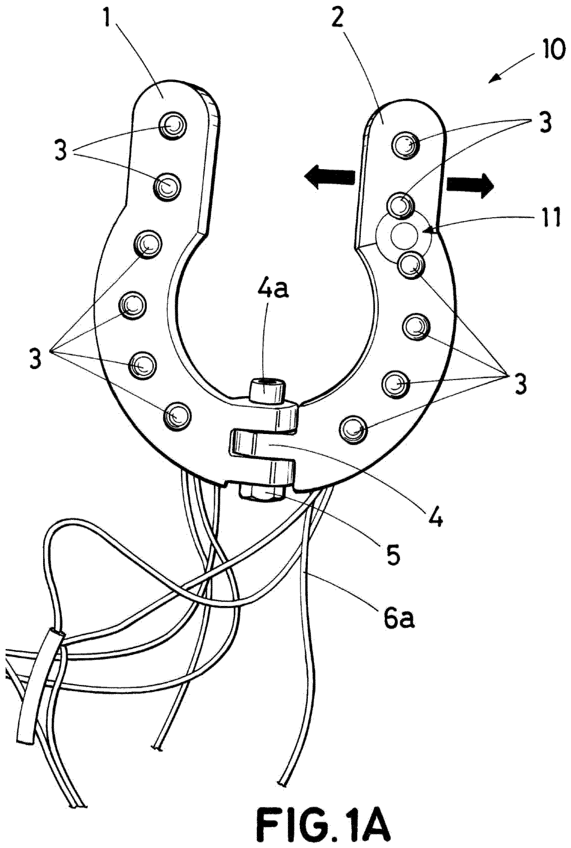

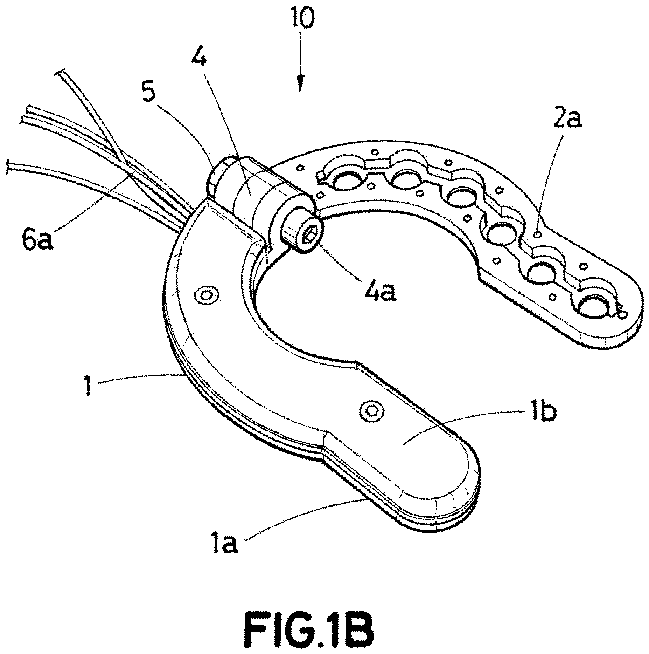

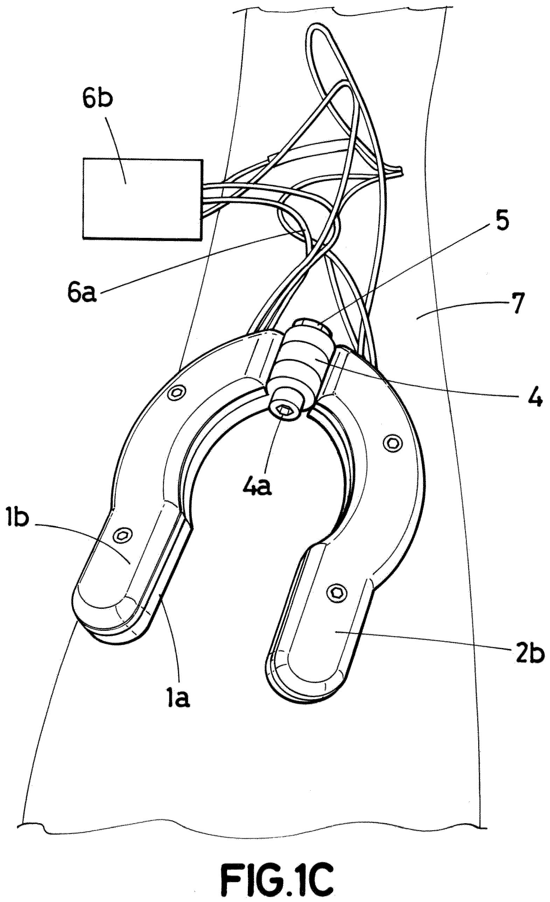

Figures 1A, 1B, and 1C show a first embodiment of a vein transilluminator 10 according to the present invention.

In this embodiment of the invention, the vein transilluminator 10 comprises two arms 1 and 2, each of said arms being formed by joining two different parts: a base 1a (2a in the case of the second arm) and a cover 1b (2b in the case of the second arm).

The LEDs 3 are housed in the bases 1a and 2a of the arms 1 and 2, whereas the covers 1b and 2b are provided with holes that allow said LEDs 3 to illuminate the part of the patient's body that is the object of the medical intervention or test.

The arms 1 and 2 are joined together by means of a joint 4 which, in this particular embodiment of the invention, comprises a threaded bolt 4a that serves as a rotating shaft. In turn, the locking means comprise a nut 5 that allows the bolt 4a to be tightened so as to prevent the movement of the arms 1 and 2.

Likewise, the electrical power supply means comprise a plurality of cables 6a connecting electric batteries, housed inside an external box 6b, with the LEDs 3. Furthermore, said external box 6b has an integrated electrical switch that allows or prevents, as desired, the passage of electrical current from the batteries to the LEDs 4 through the cables 6a, as well as electronic control means required for correct operation such as, for example: light intensity regulators, internal battery charging electronics, etc.

The fact that the electrical power supply means are made up of portable electronics allows use thereof in medical interventions in which it is not comfortable or feasible to keep the device connected to the electrical grid. The low electrical consumption of the LEDs and the progress in the development of high-capacity rechargeable batteries allow this greater versatility of the new device.

In the embodiment envisaged in Figure 1A, it can be seen that at least one of the arms 1-2 is provided with a secondary joint 11 configured in such a way as to allow changing the relative position of one arm (1) with respect to the other arm (2) in the same plane, which offers the specialist the possibility of additionally varying the geometry of the transilluminator 10, according to the area of the patient where it is applied, achieving greater adaptability and amplitude of the working field, as well as facilitating use thereof in combination with other examination means such as, for example, an Eco-Doppler probe.

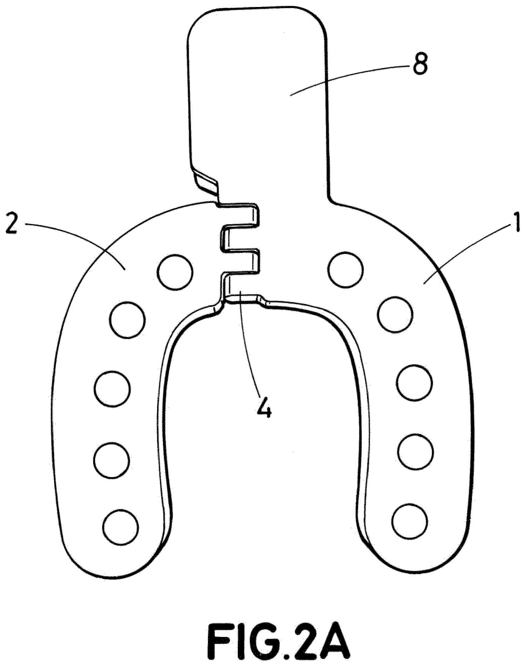

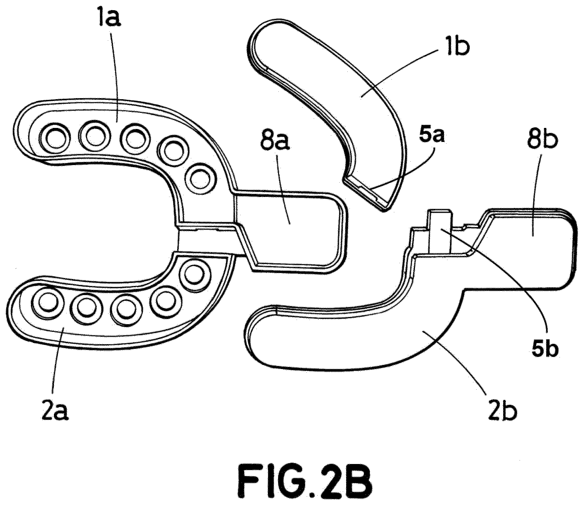

Figures 2A and 2B show a second embodiment of a vein transilluminator 10 according to the present invention.

As in the preceding case, in this second embodiment, the vein transilluminator 10 comprises two arms 1 and 2, each of said arms being formed by joining two different parts: a base 1a (2a in the case of the second arm) and a cover 1b (2b in the case of the second arm).

The bases 1a and 2a of the arms 1 and 2 are intended for housing the LEDs 3, whereas the covers 1b and 2b are provided with holes that allow said LEDs 3 to illuminate the part of the patient's body that is the object of the medical intervention or test.

In this embodiment of the invention, the body of the transilluminator further comprises a housing 8 formed by a cover 8a and a base 8b, which is intended for housing the batteries of the electrical power supply means and the control electronics. Alternatively, said housing 8 may also be intended for housing alternative locking means 5a and 5b for locking the joint 4.

The present invention is in no way limited to the embodiments disclosed herein. Other possible different embodiments of this invention will be apparent to the person skilled in the art in view of the present description. Accordingly, the scope of protection of the present invention is defined exclusively by the following claims.

## Claims

1. A vein transilluminator (10) comprising:
- a body formed by two arms (1, 2);
- a plurality of LEDs (3) housed in said arms (1, 2);
- electrical power supply means (6a, 6b) configured to electrically power the LEDs (3);
**characterised in that** the arms (1, 2) are joined together by means of at least one joint (4), said joint (4) being configured in such a way as to allow said arms (1, 2) to be arranged in two different planes.

2. The vein transilluminator (10) according to claim 1, wherein the LEDs (3) have a correlated color temperature in the range of 2700 - 3500 K.

3. The vein transilluminator (10) according to any of the preceding claims, wherein the electrical power supply means (6a, 6b) comprise at least one electric battery and at least one cable (6a) electrically connecting said battery with the LEDs (3).

4. The vein transilluminator (10) according to any of the preceding claims, wherein the joint (4) is further provided with locking means (5, 5a, 5b).

5. The vein transilluminator (10) according to any of the preceding claims, wherein at least one of the arms (1, 2) is provided with a secondary joint (11) configured in such a way as to allow varying the relative position of one arm (1) with respect to the other arm (2) in the same plane.
